# EUROPEAN PATENT SPECIFICATION

(11) **EP 2 953 679 B1**
(45) Date of publication and mention of the grant of the patent: **17.06.2020**
(21) Application number: 14749385.2
(22) Date of filing: 04.02.2014
(51) Int. Cl.: A61N 1/36, H04R 25/00

(54) **FITTING UNILATERAL ELECTRIC ACOUSTIC STIMULATION FOR BINAURAL HEARING**
ANPASSUNG EINER EINSEITIGEN ELEKTROAKUSTISCHEN STIMULATIONSVORRICHTUNG FÜR BEIDSEITIGES HÖREN
RÉGLAGE D'UNE STIMULATION ÉLECTROACOUSTIQUE UNILATÉRALE POUR AUDITION BINAURALE

(30) Priority: 05.02.2013 US 201361761063 P
(43) Date of publication of application: 16.12.2015
(73) Proprietor: MED-EL Elektromedizinische Geräte GmbH, 6020 Innsbruck (AT)
(72) Inventor: POLAK, Marek, 6020 Innsbruck (AT)
(74) Representative: Lucke, Andreas
(86) International application number: PCT/US2014/014648
(87) International publication number: WO 2014/123890

(56) References cited:
- WO-A1-92/08330
- US-A- 5 870 481
- US-A1- 2004 172 101
- US-A1- 2006 287 690
- US-A1- 2006 287 690
- US-A1- 2010 198 300
- US-A1- 2010 280 307
- US-A1- 2012 224 705
- US-A1- 2013 004 000
- US-B2- 8 295 497
- CHING T Y C ET AL: "Binaural-bimodal fitting or bilateral implantation for managing severe to profound deafness: a review", TRENDS IN AMPLIFICATION, SAGE, US, vol. 11, no. 3, 1 September 2007 (2007-09-01), pages 161-192, XP008141577, ISSN: 1084-7138, DOI: 10.1177/1084713807304357

## Description

### Cross-Reference to Related Applications

This application claims priority from United States provisional patent application serial number 61/761,063, filed February 5, 2013, entitled "Unilateral Fitting Process In Electric Acoustic Stimulation In Patients With Binaural Hearing".

### Background Art

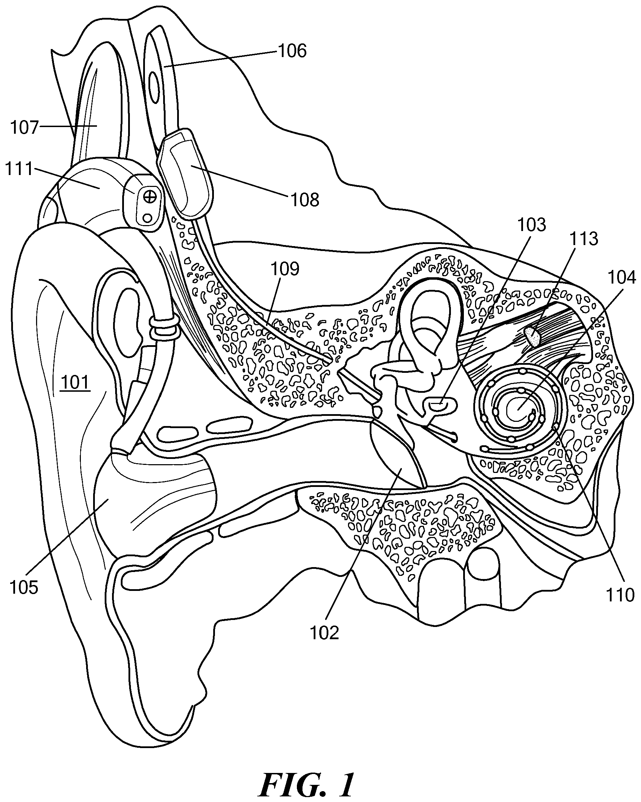

A normal ear transmits sounds as shown in Figure 1 through the outer ear **101** to the tympanic membrane **102** which moves the bones of the middle ear **103** (malleus, incus, and stapes) that vibrate the oval window and round window membrane openings of the cochlea **104.** The cochlea **104** is a long narrow duct wound spirally about its axis for approximately two and a half turns in a human cochlea. It includes an upper channel known as the scala vestibuli and a lower channel known as the scala tympani, which are connected by the cochlear duct. The axial center of the cochlea **104** is called the modiolar where the spiral ganglion cells of the acoustic nerve **113** reside. In response to received sounds transmitted by the middle ear **103,** the fluid-filled cochlea **104** functions as a transducer to generate electric pulses which are sensed by the acoustic nerve **113** and sent to the brain.

Hearing is impaired when there are problems in the ability to transduce external sounds into meaningful action potentials along the neural substrate of the cochlea. To improve impaired hearing, hearing prostheses have been developed. For example, when the impairment is related to operation of the middle ear, a conventional hearing aid may be used to provide acoustic-mechanical stimulation to the auditory system in the form of amplified sound to the tympanic membrane. Or when the hearing impairment is associated with the cochlea, a cochlear implant with an implanted electrode carrier can electrically stimulate adjacent auditory neural tissue with small currents.

In some patients with some residual hearing in the lower acoustic frequencies, a conventional hearing aid and a cochlear implant can be combined together in a hybrid Electric Acoustic Stimulation (EAS) system. The hearing aid acoustically amplifies lower acoustic frequencies perceived by human ear, while the cochlear implant electrically stimulates the middle and high frequencies. *See* von Ilberg et al, Electric-Acoustic Stimulation of the Auditory System, ORL 61:334-340; Skarzynski et al, Preservation of Low Frequency Hearing in Partial Deafness Cochlear Implantation (PDCI) Using the Round Window Surgical Approach, Acta OtoLaryngol 2007;127:41-48; Gantz & Turner, Combining Acoustic and Electrical Speech Processing: Iowa/Nucleus Hybrid Implant, Acta Otolaryngol 2004;124:344-347; Gstöttner et al., Hearing Preservation in Cochlear Implantation for Electric Acoustic Stimulation, Acta Otolaryngol 2004; 124:348-352.

Figure 1 also shows some components of a typical EAS system which includes an external microphone that provides an acoustic signal input to an external signal processor **111** where two different signal processing paths are developed. An upper acoustic frequency range communications signal containing middle and high frequency range acoustic is converted into a digital data format, such as a sequence of data frames, for transmission via a transmitter coil **107** over a corresponding implanted receiver coil **106** into the electric implant **108.** Besides receiving the processed acoustic information, the electric implant **108** also performs additional signal processing such as error correction, pulse formation, etc., and produces an electric stimulation pattern (based on the extracted acoustic information) that is sent through an electrode lead **109** to an implanted electrode array **110.** Typically, this electrode array **110** includes multiple electrode contacts on its outer surface that provide selective electric stimulation of the cochlea **104.** The external signal processor **111** also creates a lower acoustic frequency range communications signal to a conventional hearing aid **105** in the ear canal which acoustically stimulates the tympanic membrane **102,** and in turn the middle ear **103** and cochlea **104.**

To achieve optimal hearing preservation outcomes in a large population of EAS patients, a controlled electrode insertion depth was used (e.g., 18-22 mm in order to reach 360 degree of electrode insertion). More particularly, by investigating optimal electrodes, surtical approaches and how deep the electrode can be inserted in order to minimize electrode insertion trauma, higher hearing preservation rates have been achieved. The ultimate goal is the maximum possible electrode insertion depth while preserving acoustic hearing in as broad population of cochlear implantees as possible.

Following surgical implantation, the hearing aid and/or cochlear implant of the EAS subject must be custom fit to optimize its operation with the specific patient user. For the fitting process, it is important, for example, to know if an audible percept is elicited and how loud the percept is. Normally this information is gained using behavioral measures. For example, for each electrode contact the CI user may be asked at what stimulation level the first audible percept is perceived (hearing threshold (THR)) and at what stimulation level the percept is too loud (maximum comfort level (MCL)).

It is of high importance to develop a fitting algorithm for EAS subjects. The individual fitting techniques for the hearing aid and the speech processor of a cochlear implant for electrical stimulation is well described in numerous papers and patents. However, having the combination of both systems on the implanted side, a simple combination of both fitting techniques is not easily achieved. Studies show that separate fittings for the acoustic and electrical stimulations does not lead to an optimized benefit for EAS subjects (*See* Polak M., Lorens A., Helbig S., McDonald S., McDonald S., Vermeire K., Fitting of the Hearing System Affects Partial Deafness Cochlear Implant Performance, Cochlear Implants International, Vol. 11 Supplement 1, June, 2010, 117-21; and Nopp P. and Polak M., From Electric Acoustic Stimulation to Improved Sound Coding in Cochlear Implants, Van de Heyning P., Kleine Punte A. (eds), Cochlear Implants and Hearing Preservation. Adv Otorhinolaryngol, Basel, Karger, 2010, Vol. 67:88-95). Polak et al. (2010) shows that a single parameter change (e.g., lower frequency end of electrical stimulation, AGC compression, AGC threshold, and lower cut slope) may have a dramatic effect on the benefit of EAS patients for hearing in quiet and/or noisy environments.

Acoustic and electric optimized fitting parameters depend on the level of hearing preserved and preoperative residual hearing. Consequently, it is disadvantageous that the hearing aid component and CI component of the combined audio processor be fitted separately.
US 2010/280307 A1 discloses a method for fitting a bimodal stimulating medical device comprising a bimodal cochlear implant system and a fitting system that may be used in fitting devices to a recipient to be fitted with a bilateral hearing system.
US 2006/287690 A1 describes a bimodal hearing prosthesis capable of applying acoustic stimulation and electrical stimulation to a cochlea at a controlled time relative to each other. The bimodal hearing prosthesis comprises a loudspeaker for acoustically stimulating the cochlear and a stimulator unit for electrically stimulating the cochlea.
In the article "Binaural-bimodal fitting or bilateral implantation for managing severe to profound deafness: a review" by Ching T. Y. C. et al (Trends in amplification, Sage, US, vol. 11, no. 3, 1 September 2007, pages 161 to 192, ISSN 1084-7138) two methods of binaural hearing, binaural/bimodal fitting combining a cochlear implant and a hearing aid at opposite ears, and bilateral implantation of two cochlear implants are discussed to address the question of how binaural hearing can be better achieved.

### Summary of the Embodiments

In accordance with an embodiment of the invention there is provided a method of fitting an Electric Acoustic Stimulation (EAS) system of a patient that has binaural hearing, as defined in claim 1. The patient has an ipsilateral ear and a contralateral ear opposite the ipsilateral ear, with the EAS system associated with the ipsilateral ear of the patient. The method includes developing stimulation parameters of the EAS system unilaterally while taking into account hearing abilities of the contralateral ear. The EAS system is configured based on the developed stimulation parameters.

In accordance with related embodiments of the invention, developing stimulation parameters of the EAS system may include determining frequency coverage for acoustical and/or electrical regions of the EAS system. The method may include determining a frequency range covered by acoustical residual hearing associated with the EAS system, and determining a frequency range covered by electrical stimulation associated with the EAS system. If there is a frequency gap that is not covered by either the acoustical residual hearing or the electrical stimulation of the EAS system, the method may further include developing stimulation parameters such that the frequency gap is covered by electrical stimulation. If there is overlap in frequency coverage of the acoustical residual hearing and the electrical stimulation of the EAS system, the method may further include developing stimulation parameters to decrease electrical stimulation by at least one apical electrode of the EAS system, so as to allow only acoustical stimulation in the region of usable acoustical hearing.

In accordance with further related embodiments of the invention, developing stimulation parameters of the EAS system unilaterally may include maintaining functional binaural sound processing of the patient. This may include optimizing interaural level differences (ILD) and/or optimizing interaural time delays (ITD).

In still further related embodiments of the invention, the method may include determining treatment of the contralateral ear.

In accordance with another embodiment of the invention, a system for modifying stimulation parameters of an Electric Acoustic Stimulation (EAS) system of a patient that has binaural hearing as defined in claim 5 is provided. The patient has an ipsilateral ear and a contralateral ear opposite the ipsilateral ear, with the EAS system associated with the ipsilateral ear of the patient. The system includes a controller configured to communicate with the EAS system, and to develop and set stimulation parameters of the EAS system unilaterally while taking into account hearing abilities on the contralateral side of the patient.

In accordance with related embodiments of the invention, the stimulation parameters may pertain to acoustical and/or electrical coverage of the EAS system. The controller may be further configured to determine a frequency range covered by acoustical residual hearing associated with the EAS system, and to determine a frequency range covered by electrical stimulation associated with the EAS system. If there is a frequency gap that is not covered by either the acoustical residual hearing or the electrical stimulation of the EAS system, the controller may be configured to develop stimulation parameters of the EAS system such that the frequency gap is covered by electrical stimulation. If there is overlap in frequency coverage of the acoustical residual hearing and the electrical stimulation of the EAS system, the controller may be configured to develop stimulation parameters of the EAS system so as to decrease electrical stimulation of at least one apical electrode of the EAS system, thereby allowing only acoustical stimulation in the region of usable acoustical hearing.

In accordance with related embodiments of the invention, the controller may be configured to develop stimulation parameters of the EAS system unilaterally so as to maintain functional binaural sound processing of the patient. The controller may be configured to develop stimulation parameters of the EAS system unilaterally so as to optimize interaural level differences (ILD and/or interaural time delays (ITD).

In accordance with further related embodiments of the invention, the controller may be configured to determine treatment of the contralateral ear. Such treatment may include a hearing aid, an EAS system, and/or a cochlear implant associated with the contralateral ear, wherein the controller may be configured to communicate with the hearing aid, the EAS system, and/or the cochlear implant.

Also disclosed herein is a system for modifying stimulation parameters of an Electric Acoustic Stimulation (EAS) system of a patient that has binaural hearing is provided. The patient has an ipsilateral ear and a contralateral ear opposite the ipsilateral ear, the EAS system associated with the ipsilateral ear of the patient. The system includes means for developing parameters of the EAS system unilaterally while taking into account hearing abilities of the contralateral ear, and means for configuring the EAS system with the developed stimulation parameters.

The means for developing stimulation parameters of the EAS system may include means for determining coverage for acoustical and/or electrical regions of the EAS system. Determining coverage may include means for determining a frequency range covered by acoustical residual hearing associated with the EAS system, and means for determining a frequency range covered by electrical stimulation associated with the EAS system. If there is a frequency gap that is not covered by either the acoustical residual hearing or the electrical stimulation of the EAS system, the means for developing the stimulation parameters may include covering the frequency gap with electrical stimulation. If there is overlap in frequency coverage of the acoustical residual hearing and the electrical stimulation of the EAS system, the means for developing the stimulation parameters may include decreasing electrical stimulation of at least one apical electrode of the EAS system so as to allow only acoustical stimulation in the region of usable acoustical hearing.

The means for developing stimulation parameters of the EAS system unilaterally may include means for maintaining functional binaural sound processing of the patient. The means for developing stimulation parameters of the EAS system unilaterally may include means for optimizing interaural level differences (ILD) and/or interaural time delays (ITD).

The system may include means for determining treatment of the contralateral ear.

Also disclosed herein is a computer program product encoded in a non-transitory computer-readable medium for fitting an Electric Acoustic Stimulation (EAS) system of a patient that has binaural hearing is provided. The patient has an ipsilateral ear and a contralateral ear opposite the ipsilateral ear, the EAS system associated with the ipsilateral ear of the patient. The product includes program code for developing stimulation parameters of the EAS system unilaterally while taking into account hearing abilities of the contralateral ear, and program code for configuring the EAS system based on the developed stimulation parameters.

The program code for developing stimulation parameters of the EAS system may include program code for determining frequency coverage for acoustical and/or electrical regions of the EAS system. The product may include program code for determining whether there is a frequency gap that is not covered by either the acoustical residual hearing or the electrical stimulation of the EAS system, and if so, developing stimulation parameters such that the frequency gap is covered by electrical stimulation. The product may include program code for determining whether there is overlap in frequency coverage of the acoustical residual hearing and the electrical stimulation of the EAS system, and if so, developing stimulation parameters to decrease electrical stimulation by at least one apical electrode of the EAS system, so as to allow only acoustical stimulation in the region of usable acoustical hearing.

The program code for developing stimulation parameters of the EAS system unilaterally may include program code for maintaining functional binaural sound processing of the patient. For example, the product may include program code for optimizing interaural level differences (ILD) and/or interaural time delays (ITD).

The product may include program code for determining treatment of the contralateral ear.

### Brief Description of the Drawings

The foregoing features of embodiments will be more readily understood by reference to the following detailed description, taken with reference to the accompanying drawings, in which:
Figure 1 shows a typical human ear having an acoustic electric hearing implant system;
Figure 2 shows various functional blocks in a system for patient fitting of a hybrid acoustic-electrical hearing implant system, in accordance with an embodiment of the present invention;
Fig. 3 shows various logical steps that may be performed in fitting an EAS system, in accordance with an embodiment of the invention; and
Fig. 4a shows a gap between the usable acoustic residual hearing and frequency range covered by the electrode insertion according to the Greenwood scale treated by electrical stimulation, in accordance with an embodiment of the invention; and
Fig. 4b shows overlap between the usable acoustic residual hearing and frequency range covered by the electrode insertion according to the Greenwood scale treating by turning off the most apical electrodes in order to allow acoustic stimulation in the region of usable acoustic hearing, in accordance with an embodiment of the invention.

### Detailed Description of Specific Embodiments

In illustrative embodiments of the invention, an optimized Electric Acoustic Stimulation (EAS) System fitting takes into account hearing abilities of the contralateral ear to obtain equal bilateral acoustic and/or electric performance. In further embodiments, electric and acoustic hearing components of the EAS system are fitted in a coordinated manner. Details are described below.

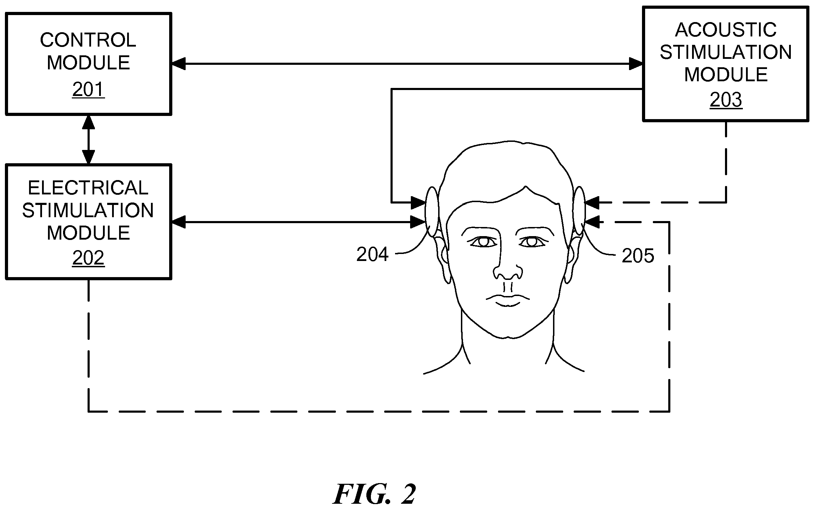

Figure 2 shows various functional blocks in a system for patient fitting of a hybrid Electric Acoustic (EAS) hearing implant system, in accordance with an embodiment of the present invention. The patient has an ipsilateral ear and a contralateral ear opposite the ipsilateral ear, with the EAS system **204** associated with the ipsilateral ear of the patient. The EAS system includes one or more audio signal processors, that may be external and/or implanted, that process an acoustic signal input so as to ultimately control acoustic amplification and/or electrical stimulation signals provided by the hearing aid and cochlear implant, respectively.

Control module **201** contains a combination of software and hardware for controlling the stimulation of electrical and acoustical pulses. For example, control module **201** may be based on a Maestro system manufactured by Med-El, Innsbruck, Austria. The electrical stimulation pulses are transmitted from the electrical stimulation module **202** (e.g., including a Device Interface Box (DIB)) to the audio prosthesis **204** which delivers them via the cochlear implant electrodes to the target nerve tissue. The control module **201** may also include software for recording near field responses from the cochlear implant electrodes. An acoustic stimulation module **203** (e.g., including a HI-PRO Box programming interface) delivers acoustic stimuli to the audio prosthesis **204** for delivery via the ear canal to the middle ear. For example, the acoustic stimulation module **203** may be an HI-PRO Box programming interface from Noah Inc. The control module may also include software and hardware for controlling the stimulation of electrical and acoustical pulses, and/or recording of responses, via, without limitation, the electrical stimulation module **202** and/or acoustic stimulation module **203,** at the contralateral ear, which may or may not include a hearing aid or implant **205.** Based on the measurements taken by the fitting system, fitting parameters of the audio processor associated with the EAS system of at least the ipsilateral ear are determined and set.

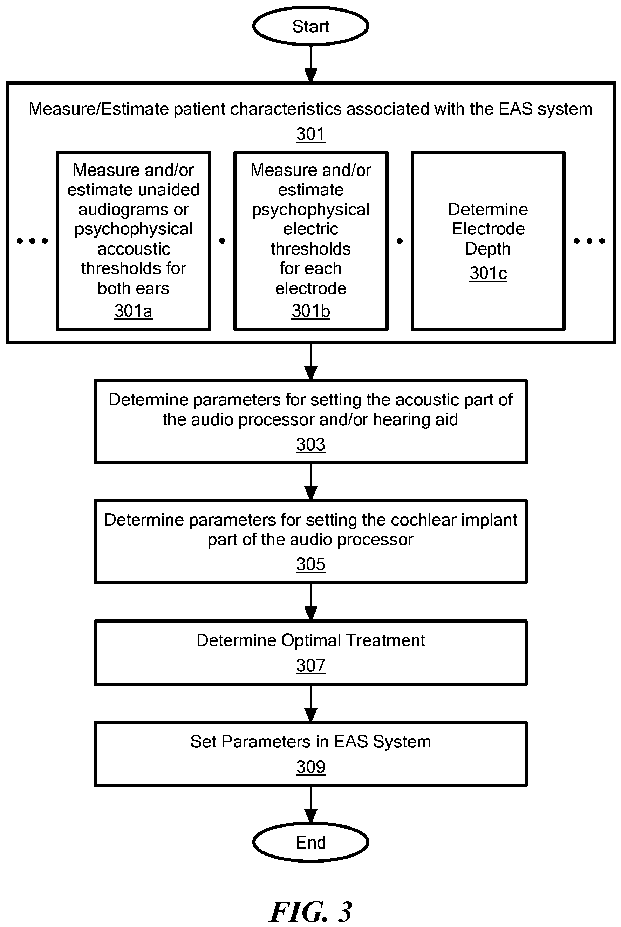

Fig. 3 shows various logical steps that may be performed in fitting an EAS system, in accordance with an embodiment of the invention. As described above, the EAS system includes both electrical stimulation via, for example, a cochlear implant, while preserving some level of natural or amplified (e.g., via a hearing aid) acoustic hearing.

The fitting advantageously may make use of contralateral non-implanted ear properties to fit the ipsilateral ear (implanted side) audio processor (referred herein as condition 1). The fitting may utilize, for example, binaural properties, such as interaural time difference (ITD) and/or and interaural level difference (ILD)) for both the ipsilateral and contralateral ear. The fitting may measure and/or determine appropriate overlap between acoustically amplified and electrically stimulated range (referred herein as condition 2). The fitting may coordinate acoustic and electric fitting parameters of the audio processor of the EAS system (referred herein as condition 3).

To begin with, various patient characteristics associated with the EAS system may be measured and/or estimated, step **301.** For example, unaided audiograms for both ears may be measured and/or estimated, and/or psychophysical acoustic thresholds determined, step **301a.** Additionally, various psychophysical electric thresholds (for example, most comfortable levels (MCLs) and/or threshold levels) for each electrode for the implanted ear may be measured and/or estimated, step **301b.** Furthermore, after placement of the electrodes into the cochlea, an electrode insertion depth may be determined, step **301c.** Electrode insertion depth may be determined, without limitation, by a postoperative CT scan. Using the Greenwood frequency scale, the determined depth may be used, without limititation, to calculate the electrical frequency coverage. For example, the insertion depth of an EAS electrode is typically controlled to a relatively shallow depth in the scala tympani which typically achieves about 360° of electrode insertion, around 18-22 mm. According to the Greenwood scale, 360° of electrode insertion in the tonotopically organized cochlea covers the acoustic frequency region from 1 kHz and higher. See Greenwood, A Cochlear Frequency-Position Function For Several Species - 29 Years Later, J Acoustic Soc Am, 1990;87(6):2592-2605. Note that where the residual acoustic hearing frequencies are lower than 1 kHz, a frequency gap arises between the highest frequency covered by residual acoustic-based hearing and the lowest frequency covered by the electrical implant. Furthermore, where the residual acoustic hearing frequencies are higher than 1 kHz, frequency overlap arises between the highest frequency covered by residual acoustic-based hearing and the lowest frequency covered by the electrical implant. It is to be understood that the various measurements and/or estimations performed in step **301** can be performed in any order.

Based on the above-described measurements and/or estimates of the patient characteristics, and under consideration of conditions 1-3, parameters for setting the acoustic part of the audio processor and/or hearing aid are determined, step **303.** These parameters may include, without limitation, number of acoustic channels, gain, channel compression, compression knee point, frequency range for acoustic amplification, and/or processing of acoustic delay, discussed in more detail below.

Additionally, based on the above-described measurements and/or estimates of the patient characteristics, and under consideration of conditions 1-3, parameters for setting the cochlear implant part of the audio processor are determined, step **305.** These parameters may include, without limitation, lower frequency end, number of deactivated most apical channels, processing of electric delay, front end compression, and/or compression knee point, discussed in more detail below.

The most optimal treatment for the implanted EAS patient may also be determined based on the above-described measurements and/or estimates of the patient characteristics, step **307.** These treatments include, without limitation, in addition to the EAS system on the ipsilateral ear, the following treatments associated with the contralateral ear: a contralateral hearing aid, a contralateral EAS system, or contralateral natural hearing.

Obtaining contralateral hearing thresholds may be performed for the purposes of setting EAS parameters of the ipsilateral ear, and that of any hearing aid/implant of the contralateral ear, such that equal loudness is obtained for both ears. This may advantageously improve localization and speech performance in real-live environments, and in particular, noisy environments.

In illustrative embodiments of the invention, the above-described methodology maintains functional binaural sound processing (allowing for processing of interaural time difference (ITD) and interaural level difference (ILD) processing) for both ears, thus allowing for the better use of binaural cues. This may include controlled loudness perception in a wide dynamic range and time delay in both ears. In general, better precision may be achieved by obtaining complex information about the hearing. This may include measurement of first hearing threshold, most comfortable levels (MCL) and uncomfortable levels for each executive audiological frequency.

Once the optimal operating parameters for the ipsilateral EAS system are determined, they are provided to and set in the EAS system, step **309.** If the contralateral side also includes a device such as a hearing aid, cochlear implant or EAS system, these devices may also be fitted with any determined parameters. In various embodiments, the contralateral side may utilize either natural hearing or hearing amplification, and may not require an implant. Alternatively, the above-described methodology may be used in a bilateral EAS fitting. In such cases, the measurement or estimation of psychophysical electric thresholds (for example, MCLs and/or Thresholds) may be performed for both ears. The above-described methodology may ensure that both cochlear implants elicit approximately equal loudness and time delay for the entire electric frequency range.

Electric and acoustic fitting parameters of an EAS system differ from those when only a hearing aid or cochlear implant is used. In order to optimize the benefit to the patient, both the hearing aid and the cochlear implant both HA and CI should be programmed in a controlled manner, as described above. A discussion of these parameters is described below in more detail.

Fitting parameters for the acoustic part of the audio processor or hearing aid may include the following.
a. the number of acoustic channels
b. gain - Treatment for summation effect of electrical and acoustic hearing may be performed. Optimization may include achieving equal loudness on both ears, as described above, channel dependent.
c. loudness compression of the acoustic part - Generally, loudness compression includes static parameters of automatic gain control (AGC) including, without limitation, expansion threshold, linear gain region, and compression threshold. Optimization may include achieving equal loudness on both ears. In case a patient uses a contralateral hearing aid or cochlear implant for the selected frequency range, the frequency specific loudness should be equal. Compression may be dependent on the level of ipsilateral and contralateral hearing for a specific frequency.
d. frequency range for acoustic amplification - Typically, only a small acoustic and electric frequency overlap is beneficial.
e. processing delay of the acoustic part - Both acoustical and electrical hearing should be processed in a controlled manner. The processing delay of the acoustic part is necessary to adjust according to the used frequency range of the acoustic amplification, frequency range of the electric stimulation and delays of the acoustic and electric processing pathway of the audio processor. The delay between the electrical and the acoustic processing may be controlled ipsilateral and/or contralateral.

Fitting parameters for the electrical stimulation part of the audio processor (e.g., speech processor of a cochlear implant) may include the following.
a. lower frequency end - Only small acoustic and electric frequency overlap is beneficial. In general, the lower frequency end may be obtained from the unaided audiogram at the level of 65dBHL (subjects with sky sloped audiograms). For subjects with flatter audiograms, the lower frequency end may be obtained from the unaided audiogram at the level when hearing is non-functional (may vary up to 80-90dBHL).
b. number of deactivated most apical channels
c. volume - Treatment for summation effect of Electrical and Acoustic hearing may advantageously be performed.
d. processing delay
e. front-end compression - in case a patient uses contralateral hearing aid or cochlear implant for the selected frequency range, the frequency specific loudness should be equal.

Setting of the AGC static parameters and acoustic gain will now be described in more detail. The static parameters of AGC may include, without limitation, expansion threshold, linear gain region, compression threshold and gain. Based on unaided audiograms, the values for these static parameters may be determined.

The static AGC parameters may be defined based on the hearing loss for each specific audiometric frequency or respective channel for both ears. For example, for 30dB Hearing Loss, the compression rate may be 1:1, with the compression rate linearly increasing with the hearing loss. Illustratively, the compression rate for 80dB HL may be 1:2.

Especially in a case when the contralateral ear does not have any functional residual hearing and the patient is not implanted with any cochlear implant, a number of compression formulas may be provided to a patient, from which the patient may then choose

(e.g., 3 different compression formulas: low (up to 1:1,5), medium (up to 1:2) and high compression (up to 1:3). Compression threshold may increase linearly from a certain hearing loss level. For example, from 70dB Hearing Loss the compression threshold may increase from, without limitation, 50dB to 70dB up to the level of hearing loss of 95dB Hearing Loss.

The determination of gain may be closely related to the chosen formulas for the compression. For each compression formula, a different formula for gain may be utilized, and in general, the gain may linearly increase with the increasing hearing loss for a particular channel.

For example, a lower gain may be associated with the low compression formula. Illustratively, for 20dB Hearing Loss, the gain may be 0 dB, and for 80dB Hearing Loss, the gain may be 40dB. For the high compression formula, the gain may be set higher. For example, for 20dB Hearing Loss, the gain may be 0dB, and for 80dB Hearing Loss, the gain may be 50dB.

Generally, if residual hearing is poor (approximately 80-90dB Hearing Loss), the acoustic gain may be set to high values (for example, a gain up to 50-60dB). Alternatively, if residual hearing is relatively good (approximately 30-20dB Hearing Loss), the acoustic gain may be set to low values. In the event hearing is normal or near to normal (approximately 0 up to 20-30dB) for a certain channel, no amplification may be performed.

In case of usable but impaired contralateral low frequency hearing, both ears may be adjusted with acoustic amplification. Especially in cases when both ears clearly differ in the amount of usable hearing and both ears use different hearing aids, the testing of equal loudness between both ears may be of particular complexity/concern. In such cases, the same hearing aids or hearing aids with similar processing algorithm may be utilized so as to maintain equal parameters. This may include amount of gain, number of amplified channels, specific aided audiogram and/or acoustic delay.

In various embodiments, the acoustically poorer ear should be amplified in order to match the better ear (i.e., the contralateral non-implanted ear). The equal loudness should be achieved as equally as possible for as wide dynamic range as possible, especially in the speech perception area or area with the highest sensitivity (approximately 50-80dB Hearing Loss). If equal hearing aids or hearing aids with similar processing algorithms are utilized, the loudness may be easily equalized for a wide dynamic range, for example, 20 to 120dB SPL. In case of normal or near-to-normal low frequency hearing, no acoustical amplification setting may be necessary.

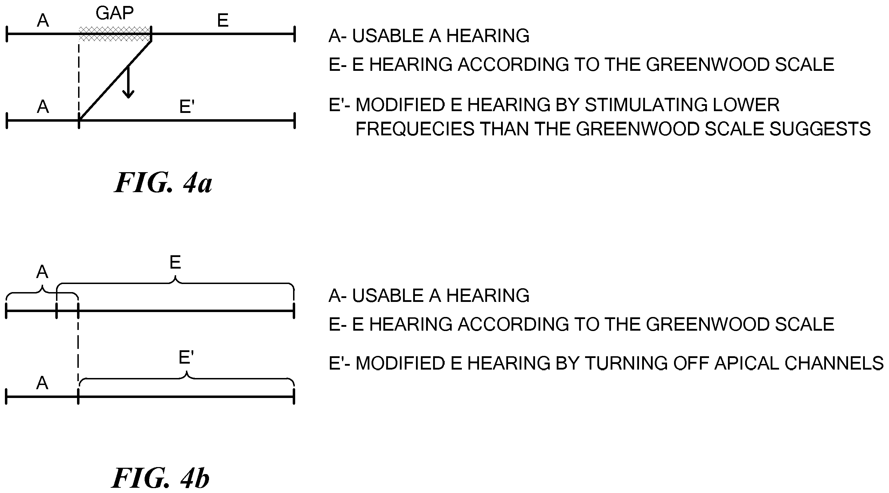

Upon measuring the usable acoustic hearing and electrode insertion depth (which, as described above, may be then further used to calculate the frequency range covered by electrical stimulation), a frequency gap or alternatively, a frequency overlap, between acoustic hearing and electrical stimulation for a given patient may be determined. Any remaining gap between the usable acoustic residual hearing and frequency range covered by the electrode insertion according to the Greenwood scale may be treated by electrical stimulation, as shown in Fig. 4a, in accordance with an embodiment of the invention. In case of overlap between the usable acoustic residual hearing and frequency range covered by the electrode insertion according to the Greenwood scale, the most apical electrodes can be turned off in order to allow acoustic stimulation in the region of usable acoustic hearing, as shown in Fig. 4b, in accordance with an embodiment of the invention. Again, electrical stimulation is used in the region with no useable acoustic hearing.

Embodiments of the invention may be implemented in whole or in part in any conventional computer programming language. For example, preferred embodiments may be implemented in a procedural programming language (*e.g.,* "C") or an object oriented programming language (*e.g*., "C++", Python). Alternative embodiments of the invention may be implemented as pre-programmed hardware elements, other related components, or as a combination of hardware and software components.

For example, a pseudo code representation of a generic embodiment might be set forth as follows:
**Process** PatientFitting
**measure and/or estimate** patient characteristics associated with the EAS system, including both ipsilateral and contralateral ear
**determine** parameters for setting the acoustic part of the audio processor and/or hearing aid
**determine** parameters for setting the cochlear implant part of the audio processor
set operating parameters of the EAS system

Embodiments can be implemented in whole or in part as a computer program product for use with a computer system. Such implementation may include a series of computer instructions fixed either on a tangible medium, such as a computer readable medium (*e.g.,* a diskette, CD-ROM, ROM, or fixed disk) or transmittable to a computer system, via a modem or other interface device, such as a communications adapter connected to a network over a medium. The medium may be either a tangible medium (*e.g.,* optical or analog communications lines) or a medium implemented with wireless techniques (*e.g.,* microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g.,* shrink wrapped software), preloaded with a computer system (*e.g.,* on system ROM or fixed disk), or distributed from a server or electronic bulletin board over the network (*e.g.,* the Internet or World Wide Web). Of course, some embodiments of the invention may be implemented as a combination of both software (*e.g.,* a computer program product) and hardware. Still other embodiments of the invention are implemented as entirely hardware, or entirely software (*e.g.,* a computer program product).

Although various exemplary embodiments of the invention have been disclosed, it should be apparent to those skilled in the art that various changes and modifications can be made which will achieve some of the advantages of the invention without departing from the true scope of the invention.

## Claims

1. A method of fitting an Electric Acoustic Stimulation, EAS, system of a patient that has binaural hearing, the patient having an ipsilateral ear and a contralateral ear opposite the ipsilateral ear, the EAS system associated with the ipsilateral ear of the patient, the contralateral ear not including a hearing aid or implant, the method comprising:
developing stimulation parameters of the EAS system unilaterally while taking into account natural hearing abilities of the contralateral ear, wherein developing the stimulation parameters includes measuring and/or estimating an unaided audiogram to obtain contralateral hearing thresholds for the contralateral ear, and developing said stimulation parameters based on said contralateral ear hearing thresholds such that equal loudness is obtained for both ears; and
configuring the EAS system based on the developed stimulation parameters.

2. The method according to claim 1, wherein developing stimulation parameters of the EAS system includes determining frequency coverage for acoustical and/or electrical regions of the EAS system.

3. The method according to claim 2, further comprising:
determining a frequency range covered by acoustical residual hearing associated with the EAS system; and
determining a frequency range covered by electrical stimulation associated with the EAS system.

4. The method according to claim 3, wherein if there is a frequency gap that is not covered by either the acoustical residual hearing or the electrical stimulation of the EAS system, the method further includes developing stimulation parameters such that the frequency gap is covered by electrical stimulation, or wherein if there is overlap in frequency coverage of the acoustical residual hearing and the electrical stimulation of the EAS system, the method further includes developing stimulation parameters to decrease electrical stimulation by at least one apical electrode of the EAS system, so as to allow only acoustical stimulation in the region of usable acoustical hearing.

5. A system for modifying stimulation parameters of an Electric Acoustic Stimulation, EAS, system of a patient that has binaural hearing, the patient having an ipsilateral ear and a contralateral ear opposite the ipsilateral ear, the EAS system associated with the ipsilateral ear of the patient, the system comprising:
a controller configured to communicate with the EAS system and to develop and set stimulation parameters of the EAS system unilaterally while taking into account natural hearing abilities on the contralateral side of the patient, wherein said stimulation parameters are developed taking into account contralateral hearing thresholds obtained by measuring and/or estimating unaided audiograms of the contralateral ears such that with the such developed stimulation parameter equal loudness is obtained for both ears when the contralateral ear does not include a hearing aid or implant.

6. The system according to claim 5, wherein the stimulation parameters include stimulation parameters pertaining to acoustical and/or electrical coverage of the EAS system.

7. The system according to claim 6, where the controller is further configured to:
determine a frequency range covered by acoustical residual hearing associated with the EAS system; and
determine a frequency range covered by electrical stimulation associated with the EAS system.

8. The system according to claim 7, wherein if there is a frequency gap that is not covered by either the acoustical residual hearing or the electrical stimulation of the EAS system, the controller is configured to develop stimulation parameters of the EAS system such that the frequency gap is covered by electrical stimulation, or wherein if there is overlap in frequency coverage of the acoustical residual hearing and the electrical stimulation of the EAS system, the controller is configured to develop stimulation parameters of the EAS system so as to decrease electrical stimulation of at least one apical electrode of the EAS system, thereby allowing only acoustical stimulation in the region of usable acoustical hearing.

## Patentansprüche

1. Verfahren zum Anpassen eines EAS-Systems (Electric Acoustic Stimulation) eines Patienten, der ein binaurales Gehör hat, wobei der Patient ein ipsilaterales Ohr und ein dem ipsilateralen Ohr gegenüberliegendes kontralaterales Ohr hat, wobei das EAS-System mit dem ipsilateralen Ohr des Patienten verbunden ist, wobei das kontralaterale Ohr kein Hörgerät oder Implantat enthält, wobei das Verfahren Folgendes umfasst:
einseitiges Entwickeln von Stimulationsparametern des EAS-Systems unter Berücksichtigung der natürlichen Hörfähigkeiten des kontralateralen Ohrs, wobei das Entwickeln der Stimulationsparameter das Messen und/oder Abschätzen eines ununterstützten Audiogramms umfasst, um kontralaterale Hörschwellen für das kontralaterale Ohr zu erhalten,
und das Entwickeln der Stimulationsparameter auf der Grundlage der Hörschwellen für das kontralaterale Ohr derart, dass für beide Ohren die gleiche Lautstärke erhalten wird; und
Konfigurieren des EAS-Systems auf der Grundlage der entwickelten Stimulationsparameter.

2. Verfahren nach Anspruch 1, wobei die Entwicklung von Stimulationsparametern des EAS-Systems die Bestimmung der Frequenzabdeckung für akustische und/oder elektrische Bereiche des EAS-Systems umfasst.

3. Verfahren nach Anspruch 2, wobei das Verfahren ferner Folgendes umfasst:
Bestimmen eines Frequenzbereichs, der durch das mit dem EAS-System assoziierten akustische Restgehör abgedeckt wird; und
Bestimmen eines Frequenzbereichs, der durch die mit dem EAS-System assoziierte elektrische Stimulation abgedeckt wird.

4. Verfahren nach Anspruch 3, wobei, wenn es eine Frequenzlücke gibt, die weder durch das akustische Restgehör noch durch die elektrische Stimulation des EAS-Systems abgedeckt ist, das Verfahren ferner die Entwicklung von Stimulationsparametern dergestalt umfasst, dass die Frequenzlücke durch elektrische Stimulation abgedeckt wird, oder wobei, wenn es eine Überlappung in der Frequenzabdeckung des akustischen Restgehörs und der elektrischen Stimulation des EAS-Systems gibt, das Verfahren ferner die Entwicklung von Stimulationsparametern zur Verringerung der elektrischen Stimulation durch mindestens eine apikale Elektrode des EAS-Systems umfasst, so dass nur eine akustische Stimulation im Bereich des nutzbaren akustischen Gehörs möglich ist.

5. System zum Modifizieren von Stimulationsparametern eines EAS-Systems (Electric Acoustic Stimulation) eines Patienten, der ein binaurales Gehör hat, wobei der Patient ein ipsilaterales Ohr und ein dem ipsilateralen Ohr gegenüberliegendes kontralaterales Ohr hat, wobei das EAS-System mit dem ipsilateralen Ohr des Patienten assoziiert ist, wobei das System Folgendes umfasst:
einen Controller, der so konfiguriert ist, dass er mit dem EAS-System kommuniziert und Stimulationsparameter des EAS-Systems einseitig entwickelt und einstellt, während er die natürlichen Hörfähigkeiten auf der kontralateralen Seite des Patienten berücksichtigt, wobei die Stimulationsparameter unter Berücksichtigung kontralateraler Hörschwellen entwickelt werden, die durch Messen und/oder Schätzen von ununterstützten Audiogrammen des kontralateralen Ohrs erhalten werden, so dass mit dem so entwickelten Stimulationsparameter gleiche Lautstärke für beide Ohren erhalten wird, wenn das kontralaterale Ohr kein Hörgerät oder Implantat enthält.

6. System nach Anspruch 5, wobei die Stimulationsparameter Stimulationsparameter umfassen, die sich auf die akustische und/oder elektrische Abdeckung des EAS-Systems beziehen.

7. System nach Anspruch 6, bei dem der Controller weiter dazu konfiguriert ist,
einen Frequenzbereich zu bestimmen, der durch das mit dem EAS-System assoziierten akustische Restgehör abgedeckt wird; und
einen Frequenzbereich zu bestimmen, der durch die mit dem EAS-System assoziierte elektrische Stimulation abgedeckt wird.

8. System nach Anspruch 7, wobei, wenn es eine Frequenzlücke gibt, die weder durch das akustische Restgehör noch durch die elektrische Stimulation des EAS-Systems abgedeckt wird, die Steuerung so konfiguriert ist, dass sie Stimulationsparameter des EAS-Systems so entwickelt, dass die Frequenzlücke durch elektrische Stimulation abgedeckt wird, oder wobei, wenn es eine Überlappung in der Frequenzabdeckung des akustischen Restgehörs und der elektrischen Stimulation des EAS-Systems gibt, die Steuerung so konfiguriert ist, dass sie Stimulationsparameter des EAS-Systems so entwickelt, dass die elektrische Stimulation mindestens einer apikalen Elektrode des EAS-Systems verringert wird, wodurch nur akustische Stimulation im Bereich des nutzbaren akustischen Gehörs zugelassen wird.

## Revendications

1. Procédé d'adaptation d'un système de stimulation électroacoustique, EAS, d'un patient qui présente une audition binaurale, le patient présentant une oreille ipsilatérale et une oreille controlatérale opposée à l'oreille ipsilatérale, le système de stimulation EAS étant associé à l'oreille ipsilatérale du patient, l'oreille controlatérale n'incluant pas de prothèse auditive ou d'implant auditif, le procédé comprenant les étapes ci-dessous consistant à :
développer des paramètres de stimulation du système de stimulation EAS unilatéralement, tout en tenant compte des capacités auditives naturelles de l'oreille controlatérale, dans lequel l'étape de développement des paramètres de stimulation inclut l'étape consistant à mesurer et/ou à estimer un audiogramme non assisté en vue d'obtenir des seuils d'audition controlatérale pour l'oreille controlatérale, et à développer lesdits paramètres de stimulation sur la base desdits seuils d'audition d'oreille controlatérale de sorte qu'une intensité sonore égale est obtenue pour les deux oreilles ; et
configurer le système de stimulation EAS sur la base des paramètres de stimulation développés.

2. Procédé selon la revendication 1, dans lequel l'étape de développement de paramètres de stimulation du système de stimulation EAS inclut l'étape consistant à déterminer une couverture de fréquence pour des régions acoustiques et/ou électriques du système de stimulation EAS.

3. Procédé selon la revendication 2, comprenant en outre les étapes ci-dessous consistant à :
déterminer une gamme de fréquences couverte par l'audition résiduelle acoustique associée au système de stimulation EAS ; et
déterminer une gamme de fréquences couverte par la stimulation électrique associée au système de stimulation EAS.

4. Procédé selon la revendication 3, dans lequel, s'il existe un écart de fréquence qui n'est pas couvert soit par l'audition résiduelle acoustique, soit par la stimulation électrique du système de stimulation EAS, le procédé inclut en outre l'étape consistant à développer des paramètres de stimulation de sorte que l'écart de fréquence est couvert par la stimulation électrique, ou dans lequel, s'il existe un chevauchement dans la couverture de fréquence de l'audition résiduelle acoustique et de la stimulation électrique du système de stimulation EAS, le procédé inclut en outre l'étape consistant à développer des paramètres de stimulation pour diminuer la stimulation électrique d'au moins une électrode apicale du système de stimulation EAS, de manière à permettre la stimulation acoustique uniquement dans la région de l'audition acoustique utilisable.

5. Système destiné à modifier des paramètres de stimulation d'un système de stimulation électroacoustique, EAS, d'un patient qui présente une audition binaurale, le patient présentant une oreille ipsilatérale et une oreille controlatérale opposée à l'oreille ipsilatérale, le système de stimulation EAS étant associé à l'oreille ipsilatérale du patient, le système comprenant :
un contrôleur configuré de manière à communiquer avec le système de stimulation EAS, et à développer et définir des paramètres de stimulation du système de stimulation EAS, unilatéralement, tout en tenant compte des capacités auditives naturelles du côté controlatéral du patient, dans lequel lesdits paramètres de stimulation sont développés en tenant compte de seuils d'audition controlatérale obtenus en mesurant et/ou en estimant des audiogrammes non assistés des oreilles controlatérales, de sorte qu'avec ledit paramètre de stimulation ainsi développé, une intensité sonore égale est obtenue pour les deux oreilles lorsque l'oreille controlatérale n'inclut pas de prothèse auditive ou d'implant auditif.

6. Système selon la revendication 5, dans lequel les paramètres de stimulation incluent des paramètres de stimulation connexes à une couverture acoustique et/ou électrique du système de stimulation EAS.

7. Système selon la revendication 6, dans lequel le contrôleur est en outre configuré de manière à :
déterminer une gamme de fréquences couverte par l'audition résiduelle acoustique associée au système de stimulation EAS ; et
déterminer une gamme de fréquences couverte par la stimulation électrique associée au système de stimulation EAS.

8. Système selon la revendication 7, dans lequel, s'il existe un écart de fréquence qui n'est pas couvert soit par l'audition résiduelle acoustique, soit par la stimulation électrique du système de stimulation EAS, le contrôleur est configuré de manière à développer des paramètres de stimulation du système de stimulation EAS de sorte que l'écart de fréquence est couvert par la stimulation électrique, ou dans lequel, s'il existe un chevauchement dans la couverture de fréquence de l'audition résiduelle acoustique et de la stimulation électrique du système de stimulation EAS, le contrôleur est configuré de manière à développer des paramètres de stimulation du système de stimulation EAS de manière à diminuer la stimulation électrique d'au moins une électrode apicale du système de stimulation EAS, ce qui permet par conséquent la stimulation acoustique uniquement dans la région de l'audition acoustique utilisable.
